# EUROPEAN PATENT APPLICATION

(11) **EP 3 616 597 A1**
(43) Date of publication of application: **04.03.2020**
(21) Application number: 18792080.6
(22) Date of filing: 25.04.2018
(51) Int. Cl.: A61B 1/12, A61B 1/00, A61B 1/012, A61B 1/018, G02B 23/24

(54) **TOOL FOR MEASURING CLEANLINESS INSIDE ENDOSCOPE CHANNEL**

(30) Priority: 28.04.2017 JP 2017089311
(71) Applicant: Toray Industries, Inc., Tokyo 103-8666 (JP)
(72) Inventor: YAGI, Toshihiko, Otsu-shi Shiga 520-8558 (JP); YABUZAKI, Shoji, Tokyo 103-8666 (JP)
(74) Representative: Mewburn Ellis LLP
(86) International application number: PCT/JP2018/016872
(87) International publication number: WO 2018/199189

(57) **Abstract**

The present invention provides a tool for measuring the cleanliness of the interior of an endoscope channel, wherein the tool is for efficiently, rapidly, and reliably examining whether or not an endoscope is clean after being cleaned and sterilized. The tool for measuring the cleanliness of the interior of an endoscope channel is characterized in that a wiping cloth containing ultrafine fibers is attached to the tip of a filament having a thickness enabling the filament to be inserted into the endoscope channel.

## Description

### TECHNICAL FIELD

The present invention relates to a tool for measuring the cleanliness of the interior of an endoscope channel, wherein the tool is for inspecting whether the interior of the endoscope channel has been cleaned successfully and reliably after being cleaned and sterilized in a process of cleaning and sterilizing the endoscope.

### BACKGROUND ART

An endoscope has a tube to the tip of which a lens is attached, the tube is inserted into the interior of a patient's body, and the interior is observed directly through the eye lens, or observed as an image displayed on a monitor. There are many kinds of endoscopes, such as bronchoscopes, upper gastrointestinal endoscopes, colonoscopes, laparoscopes, cystoscopes, and arthroscopes, and these are widely used clinically in examinations, endoscopic surgeries, and the like.

Medical institutions perform examinations and the like on many patients, and accordingly, hold established procedures for effectively performing cleaning/sterilization in particular so that appliances can be used from one patient to the providing safe medical care, it is important to clean and sterilize endoscopes reliably.

Many endoscopes have a path (channel), and can be used for topical cleaning, gas or liquid injection, drug spray, suction, treatment with a special device, and the like.

Concerning the inspection of the cleanliness of such an endoscope that has been cleaned and sterilized, the following method is known.

The tip of an endoscope is inserted into a sterile test tube containing sterile physiological saline, and the sterile physiological saline in the test tube is sucked through the forceps inlet fitted with a sterile syringe. The sucked liquid is strongly blown into the lumen of the endoscope again. This operation is repeated again and again, and the resulting liquid is used as a sample. The sample is centrifuged, and the obtained precipitate is subjected to microscopic observation and/or cultivation testing.

### SUMMARY OF INVENTION

### Technical Problem

Such a conventional method involves use of liquid, and thus, requires troublesome handling. In view of this, a problem to be solved by the present invention is to provide a tool for measuring the cleanliness of the interior of an endoscope channel, wherein the tool makes it possible to efficiently, rapidly, and reliably inspect whether or not an endoscope is clean after being cleaned and sterilized.

### Solution to Problem

To solve the problem, the present invention is constituted as follows:
(1) A tool for measuring the cleanliness of the interior of an endoscope channel, characterized by having a filament and a wiping cloth containing ultrafine fibers attached to the tip of the filament.

The preferred aspects of the present invention are constituted as follows:
(2) The foregoing tool for measuring the cleanliness of the interior of an endoscope channel, characterized in that the wiping cloth is attached in bag shape.
(3) Any of the foregoing tool for measuring the cleanliness of the interior of an endoscope channel, characterized in that the wiping cloth is a rectangularly cut piece which is folded in two and is closed at the periphery thereof except an open end so as to be in bag shape.
(4) Any of the foregoing tool for measuring the cleanliness of the interior of an endoscope channe, characterized in that the wiping cloth is cleaned.
(5) The tool for measuring the cleanliness of the interior of an endoscope channel according to (3), characterized in that the closing means is welding.
(6) The tool for measuring the cleanliness of the interior of an endoscope channel according to (5), characterized in that the wiping cloth is attached with the inside thereof turned out in such a manner that the welded portion is inside.
(7) Any of the foregoing tool for measuring the cleanliness of the interior of an endoscope channel, characterized in that the wiping cloth includes the ultrafine fibers having a fineness of 0.01 to 2.0 dtex.
(8) The tool for measuring the cleanliness of the interior of an endoscope channel according to any one of claim 1 to 7, characterized in that the filament is composed of a synthetic fiber monofilament(s).
(9) The tool for measuring the cleanliness of the interior of an endoscope channel according to any one of claim 1 to 8, characterized in that the tool for measuring the cleanliness of the interior of an endoscope channel is stored in a wrapping bag.

### Advantageous Effects of Invention

The present invention makes it possible to efficiently, rapidly, and reliably inspect whether or not an endoscope is clean after being cleaned and sterilized.

### BRIEF DESCRIPTION OF DRAWINGS

Fig. 1 is an external view of an aspect of a tool for measuring the cleanliness of the interior of an endoscope channel according to the present invention.
Fig. 2 is an external view of an aspect of a bag-like wiping cloth for a tool for measuring the cleanliness of the interior of an endoscope channel according to the present invention.
Fig. 3 is an external view of another aspect of a bag-like wiping cloth for a tool for measuring the cleanliness of the interior of an endoscope channel according to the present invention.
Fig. 4 is an enlarged external view of the attached portion of a bag-like wiping cloth for a tool for measuring the cleanliness of the interior of an endoscope channel according to the present invention.

### DESCRIPTION OF EMBODIMENTS

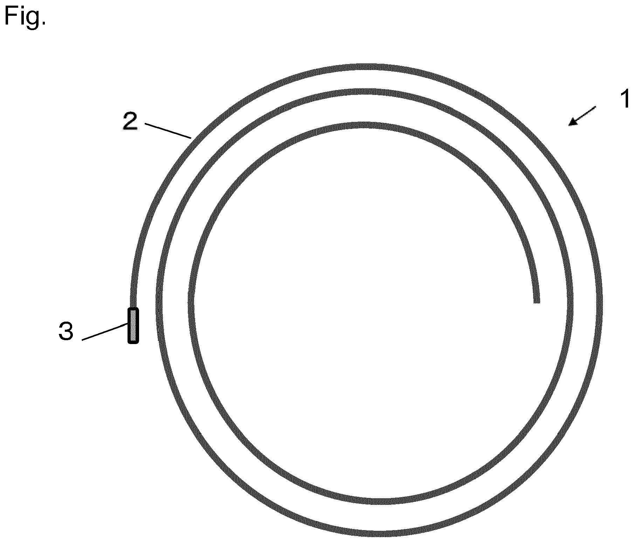

As shown in Fig. 1, a tool 1 for measuring the cleanliness of the interior of an endoscope channel according to the present invention is one in which a wiping cloth 3 containing ultrafine fibers is attached to the tip of a filament 2 having a thickness enabling the filament to be inserted into an endoscope channel.

Below, the present invention will be described in more detail.

### [Wiping Cloth]

The wiping cloth 3 used in the present invention preferably contains fibers having a single fiber fineness of 0.01 to 2.0 dtex, preferably 0.01 to 1.0 dtex. The wiping cloth more preferably contains a 0.01 to 0.5 dtex ultrafine synthetic fiber filament (A). Furthermore, the wiping cloth is preferably composed of a textile such as a knitted fabric or a woven fabric which contains not only the ultrafine synthetic fiber filament (A) but also a synthetic fiber filament (B) having a single fiber fineness of 0.8 to 3 dtex.

As below-mentioned, a wiping cloth used in the present invention preferably has an average ATP value of 45 RLU or more, and in addition, preferably achieves 90% or more as the below-mentioned ATP collection rate.

The ultrafine synthetic fiber filament (A) and the synthetic fiber filament (B) are preferably composed of a polyester fiber and/or a polyamidic fiber.

In addition, for the ultrafine synthetic fiber filament (A), a synthetic fiber that can be made ultrafine is preferably used. Examples of fibers that can be made ultrafine include composite fibers such as sea-island synthetic fibers and splittable synthetic fibers.

For the island component of a sea-island synthetic fiber, for example, a fiber that can be made ultrafine, such as a polyester fiber or a polyamidic fiber, can be used. Among others, preferable are: a polyester fiber composed of polyethylene terephthalate, polypropylene terephthalate, polybutylene terephthalate, or a copolymer thereof; and a polyamidic fiber composed of nylon 4, nylon 6, nylon 66, or a copolymer thereof. The fiber can easily be made ultrafine by dissolving the sea component in, for example, an alkaline solution.

A splittable synthetic fiber can be obtained by combining synthetic resin fibers that are the compositing components. Examples of single components include polyester fibers, polyamidic fibers, and the like. Among others, preferable are: a polyester fiber composed of polyethylene terephthalate, polypropylene terephthalate, polybutylene terephthalate, or a copolymer thereof; a polyamidic fiber composed of nylon 4, nylon 6, nylon 66, or a copolymer thereof; and combinations thereof.

In the case of a splittable synthetic fiber, treating the fiber with, for example, an alkaline liquid enables the compositing components to be separated from each other so that the fiber can be made ultrafine.

In addition, the ultrafine synthetic fiber filament (A) and the synthetic fiber filament (B) can be used in a state where they are gray yarn, or each filament can also be used after being preliminarily made into a finished yarn such as a false twist separately. Both filaments can be air-interlaced or real-twisted to be used.

Concerning the ratios of the ultrafine synthetic fiber filament (A) and the synthetic fiber filament (B) in a textile, the synthetic fiber filament (B) preferably accounts for 10 to 90 mass%, more preferably 20 to 80 mass%, still more preferably 30 to 70 mass%.

Furthermore, it is preferable that a polyester fiber is used as the ultrafine synthetic fiber filament (A), and that a polyamidic fiber is used as the synthetic fiber filament (B).

### [Filament]

The filament 2 needs to have a thickness enabling the filament to be inserted into an endoscope channel. Examples of materials include synthetic resins such as polyester, polyamide, polyolefin, polyvinyl chloride, and the like. The filament may be a multifilament, but is preferably a monofilament.

The filament has a length larger than the length of an endoscope channel, and, for example, has a length of approximately 2 m or more. As far as the thickness is concerned, it has only to be one which enables the filament to be inserted into an endoscope channel.

### [Shape of Wiping Cloth]

A bag-like wiping cloth is attached to the tip of the filament 2.

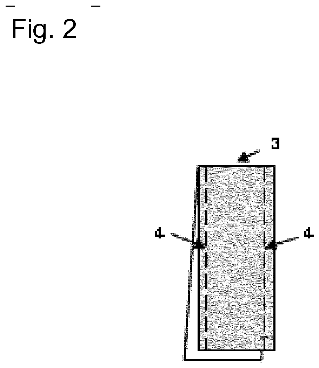

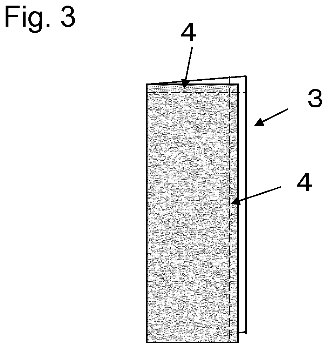

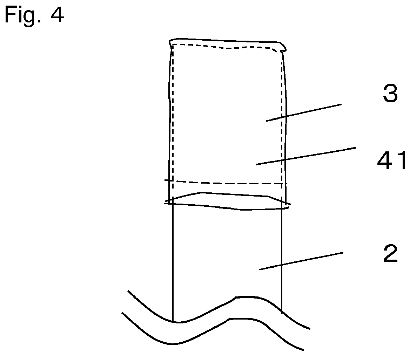

In order to form the wiping cloth in bag shape, a rectangularly cut wiping cloth is folded in two, and the upper and lower textile pieces are preferably closed by welding or bonding at the periphery except an open end so as to be formed into a shape such as a sleeping bag, as shown in Fig. 2 and Fig. 3. Fig. 2 shows a rectangular wiping cloth folded at the middle position of the longer side, and Fig. 3 shows a rectangular wiping cloth folded at the middle position of the shorter side. The welded or bonded portion lowers the wiping properties of the wiping cloth if exposed outward, and accordingly, the wiping cloth is preferably used with the inside out. As shown in Fig. 4, the bag-like wiping cloth attached to the tip of the filament 2 is further attached to the filament 2 using a means such as welding or bonding.

The width and length of the bag have only to be those which enable the bag to be inserted into an endoscope channel and enable the adhered matter in the endoscope channel to be wiped off. Examples of specific dimensional ranges include: a width of 1.2 mm to 3 mm and a length of 10 mm to 20 mm for an inside diameter ϕ of 2 mm; a width of 3 mm to 5 mm and a length of 10 mm to 20 mm for an inside diameter ϕ of 2.8 mm; a width of 4 mm to 6 mm and a length of 10 mm to 20 mm for an inside diameter ϕ of 3.2 mm; and a width of 6 mm to 8 mm and a length of 10 mm to 20 mm for an inside diameter ϕ of 3.8 mm.

In this regard, the wiping cloth is not limited to a bag shape but may be wound on the tip of the filament with part of the wiping cloth welded or bonded so that the wiping cloth can be attached to the filament.

Welding can be ultrasonic welding or high-frequency welding. In addition, bonding can be achieved using an adhesive.

In addition, the bag-like wiping cloth attached to the tip of the filament is preferably cleaned enough for measurement of cleanliness. Cleaning can be carried out by, for example, rocking in an ultrapure water bath, ultrasonic cleaning, or the like. It is also possible to use an enzymatic detergent.

### [Wrapping of Tool for Measuring Cleanliness of Interior of Endoscope Channel]

The filament 2 is long, for example, 2 m or more, and accordingly, it is preferable that each measurement tool is cleanly packed singly in coil form in a paper-made wrapping bag.

### [Wipe Test]

In the present invention, wiped matter is tested using a cleanliness measurement instrument for ATP wipe testing, "Lumitester" (registered trademark; the same applies hereinafter) PD-20, manufactured by Kikkoman Biochemifa Company and "LuciPac" (registered trademark; the same applies hereinafter) Pen that is a set including a special reagent and a swab.

This "Lumitester" PD-20 measures not only ATP (adenosine triphosphate) but also AMP (adenosine monophosphate) which results from ATP changed by heating or fermentation.

Dirt in the medical workplace is often human-derived dirt such as blood and bodily fluid, and the human-derived dirt always contains ATP and AMP in large amounts. Measuring ATP or AMP after cleaning and sterilization enables the "degree of cleaning" of organism-derived adhered matter to be known.

### [Example of Specification]

An example of the specification of a tool for measuring the cleanliness of the interior of an endoscope channel according to the present invention is shown in Table 1.

**[Table 1]**

| Subject Device | Monofilament Core (15,000 denier, 16,670 dtex) | Bag Composed of Ultrafine Fiber | Adherence between Core and "Toraysee" Bag | Cleaning Method | Wrapping |
|---|---|---|---|---|---|
| (1) Inside Diameter ϕ 2.0 mm (for transnasal endoscopy/upper) | Material: PET | MS002 Bag | ultrasonic fusion | cleaning (ultrasonic cleaning in ultrapure water bath) | cleanly packed one by one |
| | | 1.25 mm wide. Attached inside out. | | | |
| (2) Inside Diameter ϕ2.8mm (for peroral endoscopy/upper) | Outside diameter: ϕ 1.2 mm, Length: 2.5 m | Bag of "Toraysee" for CE 4.5 mm wide. Attached inside out. | | | |
| (3) Inside Diameter ϕ3.2mm (for peroral endoscopy/lower) | The curliness was corrected only for the 10 cm portion from the tip so that the portion can become straight. | Bag of "Toraysee" for CE 5.5 mm wide. Attached inside out. | | | |
| (4) Inside Diameter ϕ3.8mm (for peroral endoscopy/lower) | The tip site was not treated. | Bag of "Toraysee" for CE 7.2 mm wide. Attached inside out. | | | |

In the Table, "Toraysee" (registered trademark) for CE is a product manufactured by Toray Industries, Inc. and is produced using a knitted fabric containing a microfiber (having a single yarn fineness of 0.07 dtex). In addition, MS002 is a product produced using a microfiber (a plain-weave fabric having a single yarn fineness of 0.07 dtex).

### EXAMPLES

Below, the present invention will be described more specifically with reference to Examples.
(Examples 1 and 2 and Comparative Examples 1 and 2).

### (1) Sample

### [Example 1]

A microfiber cloth "Toraysee" (registered trademark; the same applies hereinafter) for CE, manufactured by Toray Industries, Inc., which is a knitted fabric having a single yarn fineness of 0.07 dtex was rectangularly cut. This was folded as shown in Fig. 2, the joining portions 4 were bonded by welding so as to form a bag shape, and the inside was turned out to give a bag shape again.

### [Example 2]

A synthetic fiber obtained by making a knitted fabric ("Piceme", registered trademark) ultrafine, wherein the knitted fabric is manufactured by Toray Industries, Inc. and contains a polyester fiber having a single yarn fineness of 0.26 dtex and a polyamide fiber having a single yarn fineness of 0.91 dtex. This was folded as shown in Fig. 2, the joining portions 4 were bonded by welding so as to form a bag shape, and the inside was turned out to give a bag shape again.

### [Comparative Example 1]

"LuciPac" Pen which is manufactured by Kikkoman Biochemifa Company and the raw material of which is a cotton yarn, was used.

### [Comparative Example 2]

A knitted fabric having the total fineness of 84 dtex including a single yarn fineness of 2.42 dtex. This was folded as shown in Fig. 2, the joining portions 4 were bonded by welding so as to form a bag shape, and the inside was turned out to give a bag shape once again.

### (2) ATP solution

To 200 g of sewing machine oil, TO-M1-N, manufactured by Sumico Lubricant Co., Ltd., 1 mg of disodium adenosine triphosphate (ATP crystal) manufactured by Wako Pure Chemical Industries, Ltd. was added, and the resulting mixture was exposed for three minutes to ultrasonic waves oscillated by an ultrasonic cleaner, Sanpa W-113, manufactured by Yamato Scientific Co., Ltd. to prepare a uniform ATP solution.

### (3) ATP Measurement Kit

A reagent-integrated wiping-off swab, "LuciPac" Pen, and "Lumitester" PD-20 were used, and the operation was carried out in accordance with the manual attached to the kit. The method of wetting the collecting portion of a reference sample was carried out by adding 100 µL of sterile purified water using a micropipet.

### (4) Measurement of ATP Value of ATP Solution

To a PET film sheet, 20 µL of ATP solution was added using a micropipet, and the solution was extended over a range of 30 × 30 mm using a plastic disposable loop. This portion was sampled using the "LuciPac" Pen wetted with sterile purified water, and the ATP value was measured using the "Lumitester" PD-20. Sampling with the "LuciPac" Pen was repeated five times, and the total of all ATP values was regarded as the ATP value of a blank solution that was 20 µL of the ATP solution.
The above experiment was repeated ten times, and the average value was obtained.

### (5) Measurement of Adhesion Rate of ATP to each Sample

To a PET film sheet, 20 µL of ATP solution was added using a micropipet, and the solution was extended over a range of 30 × 30 mm using a plastic disposable loop. This portion was sampled only once using the sample wetted with sterile purified water, and the ATP value was measured using the Lumitester PD-20. The same experiment was repeated ten times with each of the four samples, and the average of the ATP values of the matter wiped off with each sample was obtained. A collection rate was obtained by dividing the average of the ATP values of the ATP solution adhered to the sample by the average of the ATP values of the blank ATP solution.

Table 2 shows the resulting values (unit: RLU (Relative Light Unit)) and collection rates of the ATP sampled with the microfiber bags in Examples 1 and 2, once each, and the resulting values and collection rates of the ATP sampled, once each, with a "LuciPac" Pen cotton ball type in Comparative Example 1 and with a regular fiber type knitted fabric bag having a single yarn fineness of 2.42 dtex in Comparative Example 2.

As shown in Table 2, the ATP values were: 47,171 RLU as the average for the microfiber bag composed of a knitted fabric having a single yarn fineness of 0.07 dtex in Example 1; 45,681 RLU as the average for the microfiber bag composed of a knitted fabric containing a polyester fiber having a single yarn fineness of 0.26 dtex and a polyamide fiber of 0.91 dtex in Example 2; 34,760 RLU as the average for the "LuciPac" Pen cotton ball type in Comparative Example 1; and 28,157 RLU for the regular fiber knitted fabric bag composed of a knitted fabric having a single yarn fineness of 2.42 dtex in Comparative Example 2.

The collection rates of the ATP adhered to the respective samples were: 95.2% as the average for the knitted fabric bag having a single yarn fineness of 0.07 dtex in Example 1 according to the present invention; 92.24% for the knitted fabric bag containing a polyester fiber having a single yarn fineness of 0.26 dtex and a polyamide fiber of 0.91 dtex in Example 2; 70.19% for the "LuciPac" Pen cotton ball type in Comparative Example 1; and 56.85% for the knitted fabric bag having a single yarn fineness of 2.42 dtex in Comparative Example 2. The microfiber knitted fabric bag according to the present invention has been found to have a far better ATP adhesion rate.

**[Table 2]**

| | ATP Solution Value (Unit: RLU) | Example 1 | Comparative Example 1 | Example 2 | Comparative Example 2 |
|---|---|---|---|---|---|
| | | Knitted fabric containing fiber having a single yarn fineness of 0.07 dtex | Value of ATP sampled once with "LuciPac" Pen swab type (Unit: RLU) | Knitted fabric containing a polyester fiber having a single yarn fineness of 0.26 dtex and a polyamide fiber of 0.91 dtex | Knitted fabric having a single yarn fineness of 2.42 dtex |
| | | Value of ATP sampled once with bag-like microfiber knitted fabric (Unit: RLU) | | Value of ATP sampled once with bag-like microfiber knitted fabric (Unit: RLU) | Value of ATP sampled once with bag-like knitted fabric (Unit: RLU) |
| n=1 | 53,432 | 48,932 | 40,351 | 48,572 | 34,265 |
| n=2 | 46,123 | 44,231 | 33,062 | 45,601 | 29,145 |
| n=3 | 49,017 | 47,130 | 35,186 | 47,923 | 26,378 |
| n=4 | 48,351 | 45,285 | 33,849 | 50,662 | 25,901 |
| n=5 | 51,327 | 50,262 | 37,615 | 49,838 | 30,166 |
| n=6 | 48,026 | 46,733 | 30,621 | 43,184 | 23,087 |
| n=7 | 51,623 | 50,109 | 38,002 | 44,764 | 31,954 |
| n=8 | 49,806 | 47,108 | 37,458 | 40,719 | 28,904 |
| n=9 | 52,273 | 48,727 | 32,189 | 45,267 | 27,058 |
| n=10 | 45,283 | 43,192 | 29,267 | 40,283 | 24,715 |
| Average | 49,526 | 47,171 | 34,760 | 45,681 | 28,157 |
| ATP Collection Rate | | 95.24% | 70.19% | 92.24% | 56.85% |

These results have revealed that a measurement tool according to the present invention passed through an endoscope channel enables the matter adhered to the interior of the channel to be adhered to the measurement tool with a high probability and makes it possible to know the amount of the adhered matter more reliably.

### Reference Signs List

1: Tool for measuring the cleanliness of the interior of an endoscope channel
2: Filament
3: Wiping cloth
4: Joining portion
41: Joining portion to filament

## Claims

1. A tool for measuring the cleanliness of the interior of an endoscope channel, comprising:
a filament having a thickness enabling said filament to be inserted into said endoscope channel; and
a wiping cloth containing ultrafine fibers attached to the tip of said filament.

2. The tool for measuring the cleanliness of the interior of an endoscope channel according to claim 1, wherein said wiping cloth is attached in bag shape.

3. The tool for measuring the cleanliness of the interior of an endoscope channel according to claim 1 or 2, wherein said wiping cloth is a rectangularly cut piece which is folded in two and is closed at the periphery thereof except an open end so as to be in bag shape.

4. The tool for measuring the cleanliness of the interior of an endoscope channel according to any one of claims 1 to 3, wherein said wiping cloth is cleaned.

5. The tool for measuring the cleanliness of the interior of an endoscope channel according to claim 3, wherein the closing means is welding.

6. The tool for measuring the cleanliness of the interior of an endoscope channel according to claim 5, wherein said wiping cloth is attached with the inside thereof turned out in such a manner that the welded portion is inside.

7. The tool for measuring the cleanliness of the interior of an endoscope channel according to any one of claims 1 to 6, wherein said wiping cloth includes said ultrafine fibers having a fineness of 0.01 to 2.0 dtex.

8. The tool for measuring the cleanliness of the interior of an endoscope channel according to any one of claims 1 to 7, wherein said filament is composed of a synthetic fiber monofilament(s).

9. The tool for measuring the cleanliness of the interior of an endoscope channel according to any one of claims 1 to 8, wherein said tool for measuring the cleanliness of the interior of an endoscope channel is stored in a wrapping bag.
